(19) Europäisches Patentamt
European Patent Office
Office européen des brevets

(11) Publication number: **0 475 506 A1**

(12) **EUROPEAN PATENT APPLICATION**

(21) Application number: **91202232.4**

(51) Int. Cl.$^5$: **A61K 31/17**

(22) Date of filing: **03.09.91**

(30) Priority: **10.09.90 EP 90202397**

(43) Date of publication of application: **18.03.92 Bulletin 92/12**

(84) Designated Contracting States: **AT BE CH DE DK ES FR GB GR IT LI LU NL SE**

(71) Applicant: **DUPHAR INTERNATIONAL RESEARCH B.V**
**C.J. van Houtenlaan 36**
**NL-1381 CP Weesp(NL)**

(72) Inventor: **van Laar, Ben**
**c/o OCTROOIBUREAU ZOAN B.V., P.O. Box 140**
**NL-1380 AC Weesp(NL)**
Inventor: **Groot, Yvonne**
**c/o OCTROOIBUREAU ZOAN B.V., P.O. Box 140**
**NL-1380 AC Weesp(NL)**
Inventor: **van der Heijden, Marja**
**c/o OCTROOIBUREAU ZOAN B.V., P.O. Box 140**
**NL-1380 AC Weesp(NL)**

(74) Representative: **Breepoel, Peter M. et al**
**Octrooibureau Zoan B.V. P.O. Box 140**
**NL-1380 AC Weesp(NL)**

(54) **Pharmaceutical formulation containing a benzoyl urea derivative.**

(57) The invention is concerned with an pharmaceutical formulation containing a benzoyl urea derivative according to the formula 1

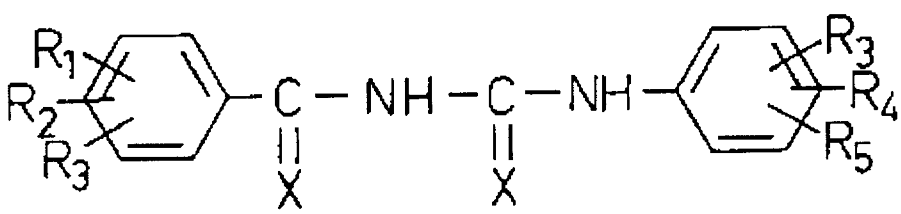

(1)

in which:

- X is an oxygen or a sulfur-atom;
- $R_1$ is a hydrogen atom, a group (0-A)n, wherein A represents a hydrogen atom, an alkanoyl group having 1 to 6 carbon atoms, a carbamoyl group optionally substituted by alkyl containing 1 to 6 carbon atoms or by phenyl, a benzylcarbonyl group optionally substituted by halogen, a benzoyl group, a benzyl group, an aryloxycarbonyl group optionally substituted by halogen, or an alkoxycarbonyl-alkyl group containing from 1 to 6 carbon atoms, or $R_1$ is an alkylmercapto group, having 1 to 3 carbon atoms or a group -S-A, wherein A has the above given meaning;
- $R_2$ is a hydrogen or halogen atom, an alkyl group having 1 to 6 carbon atoms optionally substituted by halogen, or $R_2$ is a nitro group;
- $R_3$ is a hydrogen or halogen atom, a nitro group, a 1 to 6 carbon atoms containing alkyl group optionally substituted by halogen, an alkoxy group containing 1 to 6 carbon atoms optionally substituted by halogen, a cycloalkyl group, containing 5-10 carbon atoms, optionally substituted by alkyl groups having 1-3 carbon atoms, an alkanoyl group containing from 1 to 6 carbon atoms, a benzoyl group, a nitrile group, an amino group optionally substituted by 1 or 2 alkyl groups having 1 to 3 carbon atoms, an alkoxycarbonyl group

EP 0 475 506 A1

containing from 1 to 6 carbon atoms, or a piperidinocarbonyl group;

- $R_4$ is a hydrogen atom, a group $(0\text{-}A)_m$ or S-A, wherein A has the above given meaning, or an alkylmercapto group wherein the alkyl group contains from 1 to 3 carbon atoms;
- $R_5$ is a hydrogen atom, or represents from 1 to 3 halogen atoms;
- n and m are integers, whereas the sum $n + m$ has the value from 1 to 6,

together with an ionic surface-active compound.

The present invention is concerned with pharmaceutical formulations containing a benzoyl urea derivative suitable for oral administration.

Pharmaceutical compositions containing benzoyl urea derivatives are applied e.g. as anti-tumor medicines. However, these benzoyl urea compounds generally have a low solubility in water, and hence have a low biological availability, in particular, on oral administration.

In order to improve the biological availability, formulations for oral administration of benzoyl urea compounds have been described which additionally contain non-ionic surfactants, such as a non-ionic phosphoric acid ester, a polyethylene hardened castor oil, a polyoxyethylene sorbitan fatty acid ester, a sugar ester or a polyoxyethylene polyoxypropylene block polymer (EP 0192235, EP 0233559, EP 0262560, EP 0264904, EP 0381987).

With these surfactants it was not possible to prepare suspensions of small enough particle size and adequate stability. As a result thereof the the biological availability of the active constituent was not satisfactory.

The purpose of the present invention is to provide a formulation containing a benzoyl urea compound which shows a much better biological availability of the active constituent.

To this end the formulation contains a benzoyl urea derivative described in EP 136745 and having the general formula 1

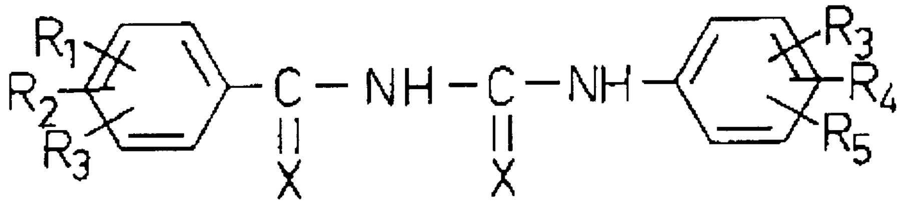

(1)

in which:

- X is an oxygen or a sulfur-atom;
- $R_1$ is a hydrogen atom, a group (0-A)n, wherein A represents a hydrogen atom, an alkanoyl group having 1 to 6 carbon atoms, a carbamoyl group optionally substituted by alkyl containing 1 to 6 carbon atoms or by phenyl, a benzylcarbonyl group optionally substituted by halogen, a benzoyl group, a benzyl group, an aryloxycarbonyl group optionally substituted by halogen, or an alkoxy carbonyl-alkyl group containing from 1 to 6 carbon atoms, or $R_1$ is an alkylmercapto group, having 1 to 3 carbon atoms or a group -S-A, wherein A has the above given meaning;
- $R_2$ is a hydrogen or halogen atom, an alkyl group having 1 to 6 carbon atoms optionally substituted by halogen, or $R_2$ is a nitro group;
- $R_3$ is a hydrogen or halogen atom, a nitro group, a 1 to 6 carbon atoms containing alkyl group optionally substituted by halogen, an alkoxy group containing 1 to 6 carbon atoms optionally substituted by halogen, a cycloalkyl group, containing 5-10 carbon atoms, optionally substituted by alkyl groups having 1-3 carbon atoms, an alkanoyl group containing from 1 to 6 carbon atoms, a benzoyl group, a nitrile group, an amino group optionally substituted by 1 or 2 alkyl groups having 1 to 3 carbon atoms, an alkoxycarbonyl group containing from 1 to 6 carbon atoms, or a piperidinocarbonyl group;
- $R_4$ is a hydrogen atom, a group $(0\text{-}A)_m$ or S-A, wherein A has the above given meaning, or an alkylmercapto group wherein the alkyl group contains from 1 to 3 carbon atoms;
- $R_5$ is a hydrogen atom, or represents from 1 to 3 halogen atoms;
- n and m are integers, whereas the sum n + m has the value from 1 to 6,

together with an ionic surface-active compound.

Suitable ionic surface-active compounds for use according to the present invention are: anionic surfactants like alkylsulphosuccinates (e.g. dioctylsulphosuccinate), alkyl sulphates (e.g. sodium or ammoniumlaurylsulphate), alkylethersulphates, alkanesulphonates and alkylbenzol-sulphonates; cationic surfactants like amine salts and quaternary ammonium salts such as benzethoniumchloride and cetyltrimethylammonium bromide, wherein the alkyl groups have 1-100 carbon atoms, preferably 1-25 carbon atoms.

The amount of benzoyl urea compound in the composition according to the invention may be up to 16% w/v of the aqueous solution.

The amount of ionic surface-active component in the composition according to the present invention may vary between 0.001 and 5% w/v of the aqueous solution.

Preferably, the alkylsulphosuccinates are present in an amount of between 0.01% and 1% w/v and the

alkylsulphates are present in an amount of between 0.0025% and 2% w/v.

Apart from the above-named pharmaceutically active constituent and the ionic surface-active compound, the formulation may also contain auxiliary constituents like suspending agents such as polyvinylpyrrolidon (PVP), gelatin, dextran, carboxymethylcellulose, mannitol, sorbitol.

Preferably, the formulation according to the invention is a suspension in water, or a freeze-dried composition resuspendable in water.

The present invention is especially suitable for formulations containing as the active component at least one of the following compounds:

* N-[[(4-chlorophenyl)amino]carbonyl]-2-(dimethylamino)-6-fluorobenzamide;
* 2-chloro-N-[[(4-chlorophenyl)amino)carbonyl]-6-(methylthio)-benzamide;
* N-[[(4-chlorophenyl)amino]carbonyl]-2-(dimethylamino)-6-methoxybenzamide;
* 2-dimethylamino-6-methoxy-N-[(4-methoxy-phenyl)amino]carbonylbenzamide.

The compositions according to the present invention can be prepared by grinding the active component in an aqueous suspension containing an ionic surface-active compound until the mean particle size of the active component is between about 1.50 and 3.25 micrometers.

The suspension can be filled into vials (to provide unit doses), and can optionally be freeze dried. The freeze dried material can be resuspended in water prior to use.

<u>EXAMPLE</u>

2.0 grams of the active compound N-[[(4-chlorophenyl)amino]carbonyl-2-(dimethylamino)-6-fluorobenzamide were mixed with 1.5 ml of a solution of 2.5 % sodiumdioctylsulphosuccinate (Na-DOSS) and 2.5 % polyvinylpyrrolidone (PVP) in water. This mixture was grinded in a vibrating ball mill during 2 hours at maximum speed. The thus formed suspension was diluted to 50.0 ml using a solution of 5 % PVP in water, filled into freeze drying vials (2.0 ml per vial) and subsequently freeze dried.

This method produced a suspension with a reproducible mean particle size of between 2.26 and 2.99 micrometer which was stable after freeze drying and which yielded a agglomerate-free suspension of the same particle size after resuspension in 1 ml of water.

The final composition of the resuspended material was:

| | |
|---|---|
| active compound | 80 mg |
| Na-DOSS | 1.5 mg |
| PVP 25 (Kollidon-25) | 98.5 mg |
| distilled water | 1.0 ml |

In parallel experiments, wherein non-ionic surface active compounds (Duphasol X and Pluronic F68) were used the resulting suspensions showed subsequent agglomerate formation, making these formulations less suitable for therapeutic applications.

Similarly, stable suspensions were prepared of the following compounds:

2-chloro-N-[[(4-chlorophenyl)amino)carbonyl]-6-(methylthio)-benzamide; mean particle size between 1.49 and 1.95 micrometers;

N-[[(4-chlorophenyl)amino]carbonyl]-2-(dimethylamino)-6-methoxybenzamide; mean particle size between 2.22 and 2.48 micrometer;

2-dimethylamino-6-methoxy-N-[[(4-methoxy-phenyl)amino]carbonylbenzamide; mean particle size between 2.54 and 3.12 micrometer.

**Claims**

**1.** Pharmaceutical formulation containing a benzoyl urea derivative according to the formula 1

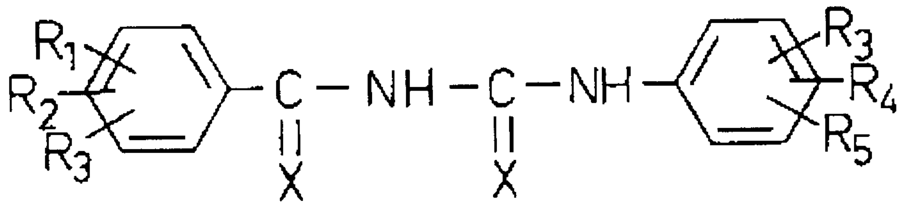

(1)

in which:

- X is an oxygen or a sulfur-atom;
- $R_1$ is a hydrogen atom, a group (0-A)n, wherein A represents a hydrogen atom, an alkanoyl group having 1 to 6 carbon atoms, a carbamoyl group optionally substituted by alkyl containing 1 to 6 carbon atoms or by phenyl, a benzylcarbonyl group optionally substituted by halogen, a benzoyl group, a benzyl group, an aryloxycarbonyl group optionally substituted by halogen, or an alkoxycarbonyl-alkyl group containing from 1 to 6 carbon atoms, or $R_1$ is an alkylmercapto group, having 1 to 3 carbon atoms or a group -S-A, wherein A has the above given meaning;
- $R_2$ is a hydrogen or halogen atom, an alkyl group having 1 to 6 carbon atoms optionally substituted by halogen, or $R_2$ is a nitro group;
- $R_3$ is a hydrogen or halogen atom, a nitro group, a 1 to 6 carbon atoms containing alkyl group optionally substituted by halogen, an alkoxy group containing 1 to 6 carbon atoms optionally substituted by halogen, a cycloalkyl group, containing 5-10 carbon atoms, optionally substituted by alkyl groups having 1-3 carbon atoms, an alkanoyl group containing from 1 to 6 carbon atoms, a benzoyl group, a nitrile group, an amino group optionally substituted by 1 or 2 alkyl groups having 1 to 3 carbon atoms, an alkoxycarbonyl group containing from 1 to 6 carbon atoms, or a piperidinocarbonyl group;
- $R_4$ is a hydrogen atom, a group $(0\text{-}A)_m$ or S-A, wherein A has the above given meaning, or an alkylmercapto group wherein the alkyl group contains from 1 to 3 carbon atoms;
- $R_5$ is a hydrogen atom, or represents from 1 to 3 halogen atoms;
- n and m are integers, whereas the sum $n + m$ has the value from 1 to 6,

together with an ionic surface-active compound.

**2.** Pharmaceutical formulation according to claim 1, wherein the benzoyl urea derivative is selected from the group consisting of:

* N-[[(4-chlorophenyl)amino]carbonyl]-2-(dimethylamino)-6-fluorobenzamide;
* 2-chloro-N-[[(4-chlorophenyl)amino)carbonyl]-6-(methylthio)-benzamide;
* N-[[(4-chlorophenyl)amino]carbonyl]-2-(dimethylamino)-6-methoxybenzamide;
* 2-dimethylamino-6-methoxy-N-[(4-methoxy-phenyl)amino]carbonylbenzamide.

European Patent Office

# EUROPEAN SEARCH REPORT

Application Number

**EP 91 20 2232**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 107 214 (DUPHAR)<br>* Abstract; claims * | 1-2 | A 61 K 31/17 |
| P,A | EP-A-0 136 745 (DUPHAR)<br>* Abstract; claims * | 1-2 | |
| A | EP-A-0 262 560 (THE GREEN CROSS CORP.)<br>* Abstract; page 5, lines 1-19; claims * | 1-2 | |

TECHNICAL FIELDS SEARCHED (Int. Cl.5)

A 61 K

The present search report has been drawn up for all claims

| Place of search | Date of completion of search | Examiner |
|---|---|---|
| The Hague | 29 November 91 | GOETZ G. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document